# EUROPEAN PATENT SPECIFICATION

(11) **EP 1 905 371 B1**
(45) Date of publication and mention of the grant of the patent: **17.06.2015**
(21) Application number: 07013118.0
(22) Date of filing: 04.07.2007
(51) Int. Cl.: A61B 18/12, A61B 18/14, A61B 18/00

(54) **Electric processing system**
Elektrisches Behandlungssystem
Système de traitement électrique

(30) Priority: 29.09.2006 US 537160
(43) Date of publication of application: 02.04.2008
(73) Proprietor: OLYMPUS MEDICAL SYSTEMS CORP., Tokyo 151-0072 (JP)
(72) Inventor: Tanaka, Kazue, Hachioji-shi Tokyo 192-8512 (JP); Irisawa, Takashi, Hachioji-shi Tokyo 192-8512 (JP); Mihori, Takashi, Hachioji-shi Tokyo 192-8512 (JP)
(74) Representative: von Hellfeld, Axel

(56) References cited:
- EP-A- 1 080 694
- US-A- 5 931 836
- US-A- 6 033 399
- US-A1- 2002 165 530
- US-A1- 2002 165 531
- US-B1- 6 398 779

## Description

The present invention relates to an electric processing system which performs a surgical procedure such as coagulation of a living tissue or sealing of a blood vessel for hemostasis by using a high-frequency power.

In general, there is known an electric surgical apparatus which uses a high-frequency power to perform a surgical procedure such as incision, coagulation or hemostasis with respect to a living tissue as typified by an electric scalpel. As a similar apparatus, there are known sealing forceps which use a high-frequency power to hermetically close and weld a blood vessel or a fibrovascular bundle. For example, in U.S. Patent No. 6033399 (U.S. Patent Application No. 08/8385458) is proposed a generator for an electric surgical apparatus (an electrosurgical generator) having an output control portion which performs feedback control by monitoring an impedance of a living tissue to desiccation the living tissue when effecting processing of sealing and welding, e.g., a part of a body cavity or a blood vessel of the living tissue and hence has an improved quality and reliability. Further, in Jpn. Pat. Appln. KOKAI Publication No. 2002-325772 is proposed an electric surgical apparatus which controls an output power or terminates processing in accordance with a change in an impedance of a living tissue based on an output time of a high-frequency power and a preset value of the impedance of the living tissue.

US 5,931,836 discloses different embodiments of an electrosurgery apparatus for treating a living tissue. According to this document, several variables can be used to determine the end of a treatment with the electrosurgery apparatus, e.g. the impedance or a phase difference between current and voltage in the case of a monopolar treatment device.

US 2002/0165531 A1 discloses an electrosurgical generator with a bipolar electrosurgical instrument. According to this document, an initial pulse is used with a high power to produce vapour bubbles to prevent current hogging. Subsequent pulses are used with lower power.

US 6,398,779 B1 discloses to use an initial low-power pulse to determine characteristics of tissue to be treated and to base the following pulse structure based on the results obtained form the initial pulse. Furthermore, this document suggests to progressively lower the voltage of pulses to avoid arcing due to the desiccation and shrinking of the tissue during treatment.

US 2002/0165530 A1 also discloses an electrosurgical device using an impedance detector to control the power used for treatment.

Bipolar type sealing forceps as one type of such apparatuses have a structure in which two distal ends (jaws) to which a high-frequency power is applied are configured into a double or single opening structure to hold a desired blood vessel or the like therebetween. These distal ends have insulating properties by using an insulating member such as ceramic or a resin to prevent an electrical short circuit from being generated between the distal ends when closed.

Since the two distal ends of the bipolar type sealing forceps respectively serve as electrodes, the forceps are used with a narrow inter-electrode distance as compared with monopolar type forceps. Therefore, when a living tissue or the like is held and a high-frequency power is applied, abnormal discharge (a spark) may occur between, e.g., edge parts of the two distal ends even in a state where the living tissue is interposed. This spark often continues when it once occurs, a fluctuation in a high-frequency power which is subjected to feedback control is also generated, and there occurs so-called chattering that impedance characteristics of a living tissue which usually rectilinearly or linearly vary fluctuate in a small range. As described above, chattering of an impedance involves a reduction in not only control over application of high-frequency power but also quality of processing, and makes a judgment upon termination of processing difficult.

The present invention provides an electric processing system which terminates abnormal discharge (extinguishes a spark) due to a high-frequency power, avoids a reduction in quality of sealing processing and can readily make a judgment upon end of processing.

The electric processing system comprises the features of claim 1.

Furthermore, the electric processing system comprises the features of claim 6.
FIG. 1 is a view showing a configuration of an electric processing system in a first embodiment according to the present invention;
FIGS. 2A, 2B, 2C, 2D, 2E and 2F are views showing output voltage characteristics and impedance characteristics obtained by the electric processing system according to the first embodiment;
FIG. 3 is a flowchart illustrating feedback control over a high-frequency power in the first embodiment;
FIG. 4 is a view showing a configuration of an electric processing system in a second embodiment according to the present invention;
FIGS. 5A, 5B, 5C, 5D, 5E and 5F are views showing output voltage characteristics and impedance characteristics obtained by the electric processing system according to the second embodiment; and
FIG. 6 is a flowchart illustrating feedback control over a high-frequency power in the second embodiment.

Embodiments according to the present invention will now be described in detail hereinafter with reference to the accompanying drawings.

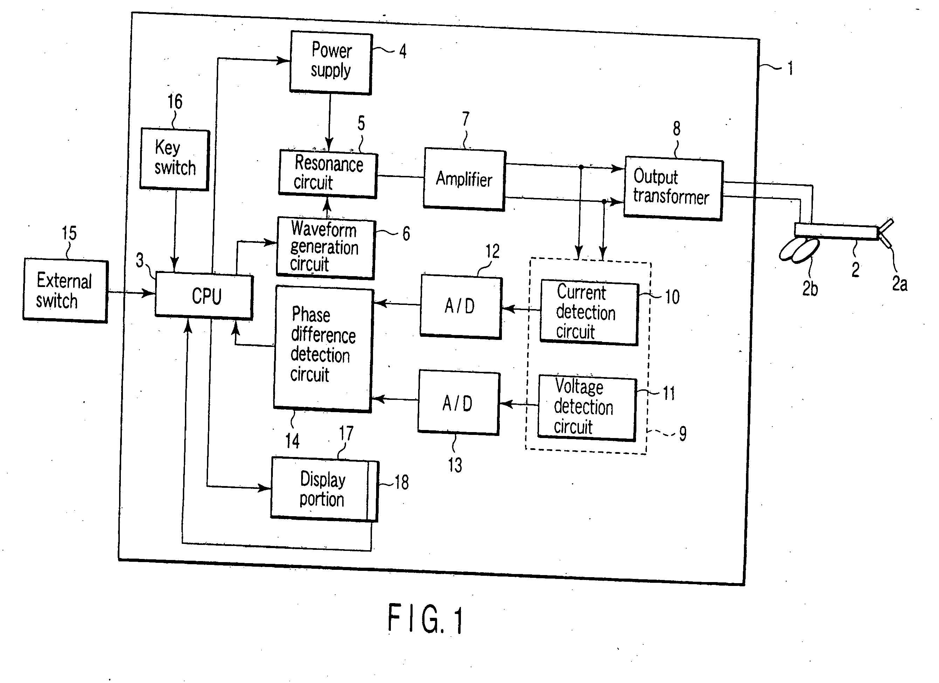

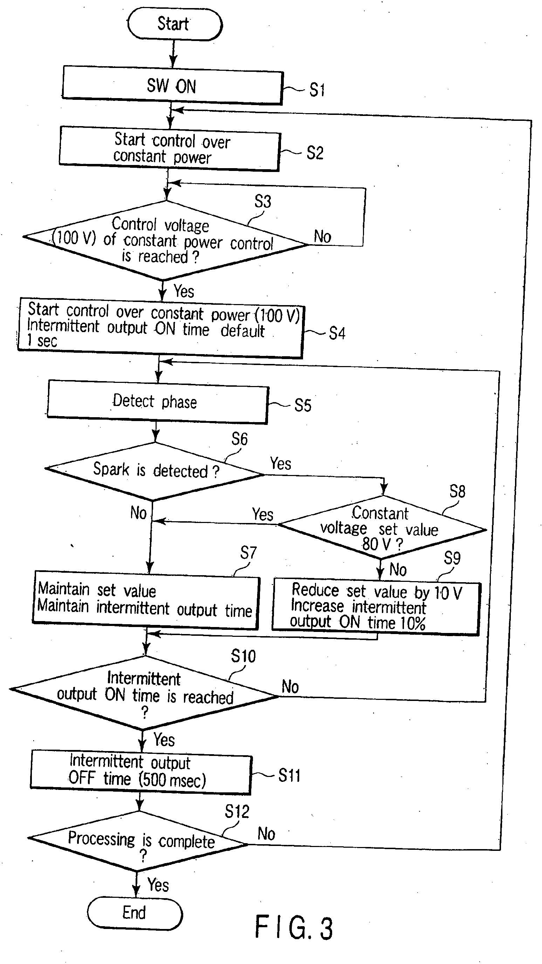

FIG. 1 shows a configuration of an electric processing system in a first embodiment according to the present invention. Moreover, FIG. 3 is a flowchart illustrating feedback control over a high-frequency power in this embodiment.

The electric processing system according to this embodiment is roughly constituted of a drive control apparatus main body 1 and an electric processing instrument, e.g., bipolar type sealing forceps 2 which perform welding processing with respect to a blood vessel or the like. Distal ends 2a in which two jaws to which a high-frequency power (a power value: a high-frequency current value x a high-frequency voltage value) can be applied are formed into a double opening or single opening structure are provided at the end of the sealing forceps 2a so that a living tissue or a blood vessel is held to perform processing such as incision, coagulation, sealing, welding or the like. An insulating member such as a ceramic member or a resin member is provided on each of the two jaws so that a non-energized condition can be attained in a closed state. Additionally, manipulating an operating portion 2b provided on an operator's hand side of the sealing forceps 2 can open/close the jaws of the distal ends 2a. It should be noted that a switch which turns application of the high-frequency power on and off may be provided in this operating portion 2b.

The drive control apparatus main body 1 is comprised of: a control portion (a CPU) 3 which controls the entire apparatus and performs feedback control over a high-frequency power applied to the sealing forceps 2; a power supply 4 which supplies a direct current; a resonance circuit 5 which converts the direct current into a high-frequency current (a high-frequency power); a waveform generation circuit 6 which controls a waveform of the high-frequency current generated by the resonance circuit 5; an amplifier 7 which amplifies the high-frequency power to a desired power value; an output transformer 8 which outputs the high-frequency power from the amplifier 7 to the jaws of the sealing forceps 2; a current/voltage detecting portion 9 consisting of a current detection circuit 10 which samples the output high-frequency power to detect a current value and a voltage detection circuit 11 which detects a voltage value; analog-to-digital converters 12 and 13 which respectively digitize the current value and the voltage value detected by the current detection circuit 10 and the voltage detection circuit 11; a phase difference detection circuit 14 which detects a change in a phase difference in detection values (the current value and the voltage value) converted into digital signals; an external switch 15 such as a hood switch which turns application of the high-frequency power on and off; a key switch (including a keyboard) 16 which is provided on the exterior of the apparatus and inputs operator instructions; a display portion 17 which displays an application state of the high-frequency voltage or information required for processing; and a touch panel switch 18 which is provided on a screen of the display portion 1 in order to input operator instructions like the key switch 16.

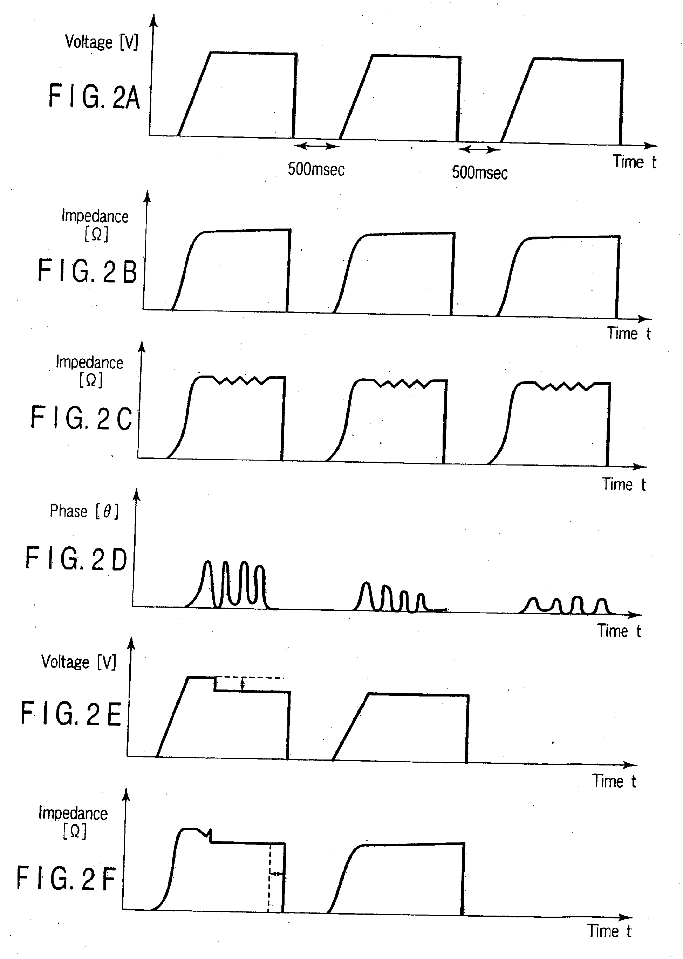

This drive control apparatus main body 1 applies a high-frequency power to the distal ends 2a of the sealing forceps 2 which hold a living tissue or a blood vessel therebetween. As shown in FIG. 2A, this high-frequency power is shaped into a pulse waveform based on an intermittent output timing that, e.g., an application time is approximately one section and a time interval of approximately 0.5 seconds is provided in such a manner that a temperature of a held living tissue or blood vessel and a temperature of a periphery of such a part are maintained at a predetermined proper temperature or below. An application state of this high-frequency power (an output value or an intermittent output timing) is appropriately changed in accordance with a design specification of the sealing forceps 2 or a state of a living tissue. An output value of the high-frequency power can be set by appropriately operating the key switch 16 or the touch panel switch 18.

It is sufficient to set an output waveform of this high-frequency power in such a manner that it becomes substantially equivalent to an amount of an applied energy based on a product of a sum of each application time of the intermittently applied high-frequency power and a high-frequency power (a high-frequency current value or a high-frequency voltage value). That is, when the high-frequency power is reduced, prolonging the application time to allow processing can suffice.

The phase difference detection circuit 14 detects a change in a phase difference in detection values (the current value and the voltage value) converted into digital signals. It is sufficient to detect a change in a phase difference between the current and the voltage to sense occurrence of abnormal discharge. It should be noted that a waveform of the high-frequency power generated in this embodiment is basically a sine waveform, and hence a zero cross circuit may be used to detect a phase signal.

FIGS. 2A to 2F show changes in a voltage waveform of the intermittently output high-frequency power, an impedance and a phase. Specifically, FIG. 2A shows a voltage waveform of a normal high-frequency power, and abnormal discharge (a spark) is not generated. FIG. 2B shows a change in an impedance of a living tissue or a blood vessel held between the distal ends 2a of the sealing forceps 2 to which the high-frequency power is applied. This change represents a normal state and corresponds to a change from several tens of ohms to approximately 500 Q, and the impedance is increased in accordance with a change in the high-frequency power and then relatively gently increased. FIG. 2C shows a change in the impedance when abnormal discharge is produced. A value of the impedance calculated based on continuously generated sparks has chattering characteristics. FIG. 2D shows a phase detection signal detected in the phase difference detection circuit 14. FIG. 2E shows output characteristics of the high-frequency power whose voltage is reduced by feedback control of the CPU 3 at the time of generation of abnormal discharge. FIG. 2F shows a change in the impedance of a held living tissue or blood vessel involved by the high-frequency power whose voltage is reduced by feedback control of the CPU 3 at the time of occurrence of abnormal discharge.

A description will now be given as to output control of the thus configured electric processing system with reference to a flowchart of FIG. 3.

First, an operator turns on the external switch 15 to start processing (step S1). Based on this on operation, application of a high-frequency power to the sealing forceps 2 is started, and feedback control begins (step S2).

Then, a judgment is made upon whether a voltage value of the high-frequency power applied to the sealing forceps 2 has reached a preset control voltage (e.g., a constant voltage having an upper limit value of 100 V) (step S3). Voltage rising in this example has a waveform shape which is slightly inclined with respect to vertical rising of a regular pulse waveform as shown in FIG. 2 in order to avoid overshoot. It should be noted that a degree of inclination of rising is appropriately set in accordance with a design specification. In this manner, a factor of a reduction in quality of processing and start of abnormal discharge due to overshoot is eliminated in the waveform. The quality of processing described herein means realization of a sealed state without carbonation of a living tissue to be sealed or adhesion in a closely-attached state without rupture in the case of a blood vessel.

When the high-frequency power has not reached the control voltage yet (NO) in the judgment at step S3, the application state is maintained. On the other hand, when the high-frequency power has reached the control voltage (YES), the control voltage is maintained and an intermittent output is applied to the sealing forceps 2 in a pulse waveform with an application time of approximately one second (step S4). At this time, an intermittent time (an output stop time) is set to approximately 500 milliseconds. Of course, this output stop time is approximately set while considering a high-frequency power value in such a manner that a temperature of a held part and a temperature of its peripheral living tissue become less than a predetermined temperature.

Subsequently, a phase difference signal detected by the phase difference detection circuit 14 is detected from an output signal of the high-frequency power (step S5). This phase difference signal represents a phase difference between a high-frequency power to be input and an input high-frequency power with a change in an impedance, and is detected by the phase difference detection circuit 14. The CPU 3 uses this phase difference detection signal to judge whether abnormal discharge (a spark) has been generated in the sealing forceps 2 (step S6). When it is determined that abnormal discharge is not generated in this judgment (NO), application is continued with the set control voltage and intermittent output timing (step S7). On the other hand, when such a phase difference detection signal as shown in FIG. 2D is detected and it is determined that abnormal discharge (a spark) has been generated by the CPU 3 (YES), a judgment is made upon whether the current control voltage (a constant voltage) has been reduced to 80 V (a judgment reference value in this embodiment) (step S8). When it is determined that the control voltage (a constant voltage) is 80 V in this judgment (YES), this 80 V is maintained and the control shifts to step S7. Further, when the control voltage is not smaller than 80 V (NO), the control voltage is reduced by 10 V, and an application time is increased by 10%. In this embodiment, in the case of the control voltage which is, e.g., 100 V, the control voltage is reduced to 90 V, and an application time of one second is prolonged to 1.1 seconds. Although a lower limit of the application voltage is set to 80 V in this embodiment, it is empirically set as a voltage which stops abnormal discharge, i.e., extinguishes a spark and enables appropriate processing. Therefore, the lower limit of the application voltage can be set to 80 V or above depending on electrical characteristics of sealing forceps 2 having a different design.

After the high-frequency power is applied at step S7, or after a reduction in the control voltage is set and the reduced voltage is applied at step S8, a judgment is made upon whether a preset application time (an intermittent output on time of approximately one second in this example) has been reached (step S10). If it is determined that the preset application time has not been reached in this judgment (NO), the control returns to step S5, and a change in an impedance is detected while maintaining application of the high-frequency power. On the other hand, if the preset application time has been reached (YES), output is stopped for the above-described output stop time of approximately 500 milliseconds (step S11).

Further, whether adhesion processing has been completed with respect to a blood vessel or the like is judged (step S12). As to a timing of completion of this adhesion processing, it is sufficient to use a known completion judgment method, e.g., an accumulated temperature (a sum of histories) of increases in temperature in a periphery of a held part, achievement of a preset impedance value at the time of completion or whether a phase difference has reached a specified value. If it is determined that welding processing has not been completed in the judgment (NO), the control returns to step S2 to continue the processing sequence. On the other hand, if the welding processing has been completed (YES), this processing sequence is terminated.

As described above, according to the first embodiment, a phase difference in the sequentially intermittently output high-frequency power is monitored for the feedback control over the high-frequency power applied to the bipolar type sealing forceps 2. When abnormal discharge (a spark) is produced at the distal ends, the high-frequency power is reduced and the application time is extended. As a result, the abnormal discharge can be terminated (the spark can be extinguished), a reduction in quality of processing can be avoided, and end of processing can be readily judged.

Furthermore, since abnormal discharge is avoided on the drive control apparatus main body 1 side in this embodiment, even if the sealing forceps 2 are changed over, the present invention can be applied by just changing a set value on the drive control apparatus main body 1 side, and the equivalent effect can be obtained, thereby providing high general versatility.

A second embodiment will now be described.

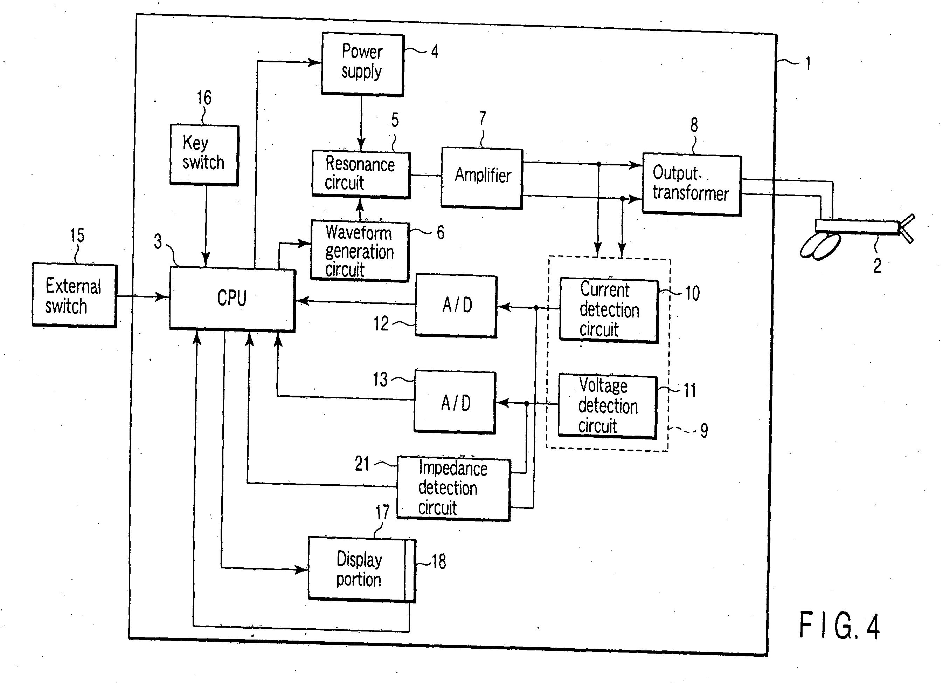

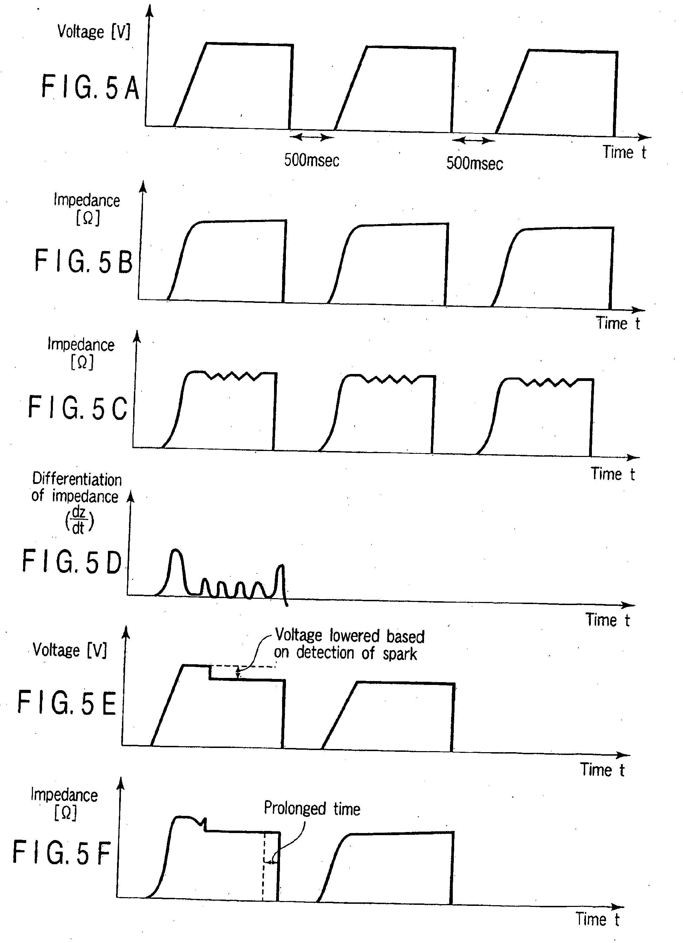

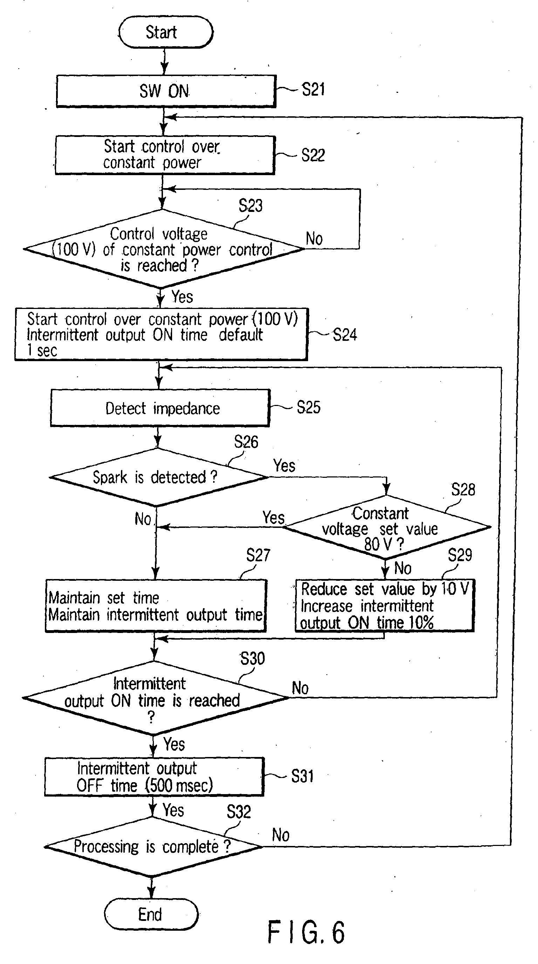

FIG. 4 shows a configuration of an electric processing system in the second embodiment according to the present invention. FIGS. 5A to 5F are views showing output voltage characteristics and impedance characteristics obtained by the electric processing system according to this embodiment. Moreover, FIG. 6 is a flowchart illustrating feedback control over a high-frequency power in this embodiment. Like reference numbers denote constituent parts in this embodiment which are equivalent to those in the first embodiment, thereby omitting a detailed description thereof.

The electric processing system according to this embodiment is constituted of a drive control apparatus main body 1 and an electric processing instrument, e.g., bipolar type sealing forceps 2 which perform welding processing with respect to a blood vessel or the like.

The sealing forceps 2 hold a living tissue or a blood vessel between distal ends 2a to carry out processing of incision and coagulation (sealing and welding) by using a high-frequency power. In this embodiment, a treatment concerning welding processing of a blood vessel or the like is performed. This embodiment provides a structure in which an impedance detection circuit 21 which detects a change in an impedance of a held living tissue based on a fluctuation in a high-frequency power is provided in place of the phase difference detection circuit 14 according to the first embodiment.

The drive control apparatus main body 1 is comprised of a control portion (a CPU) 3 which performs control over the entire apparatus and feedback control over a high-frequency power, a power supply 4, a resonance circuit 5 which generates a high-frequency power, a waveform generation circuit 6 which controls a waveform when generating a high-frequency power, an amplifier 7 which amplifies the high-frequency power, an output transformer 8 which outputs the high-frequency power to the sealing forceps 2, a current/voltage detecting portion 9 having a current detection circuit 10 and a voltage detection circuit 11, analog-to-digital converters 12 and 13 which respectively digitize a detected current value and voltage value, an impedance detection circuit 21 which samples the generated high-frequency power (a current value and a voltage value) to detect an impedance, an external switch 15 such as a hood switch, a key switch (including a keyboard) provided on the exterior of the apparatus, a display portion 17 which displays an application state of the high-frequency voltage or information required for processing, and a touch panel switch 18 which inputs operator instructions like the key switch 16.

This drive control apparatus main body 1 applies a high-frequency power to the distal ends 2a of the sealing forceps 2 which hold a living tissue or a blood vessel therebetween. As shown in FIG. 5A, this high-frequency power is shaped into a pulse waveform based on an intermittent output timing with an application time of approximately 1 second and a time interval of approximately 500 milliseconds in such a manner that a temperature of a periphery of a held living tissue or a blood vessel is maintained at a preset proper temperature or below. An application state of this high-frequency power is appropriately changed as in the first embodiment.

It is sufficient to set an output waveform of this high-frequency power in such a manner that it becomes substantially equivalent to an amount of an applied energy based on a product of a sum of each application time of the intermittently applied high-frequency power and a high-frequency power value (a high-frequency current value or a high-frequency voltage value). That is, when the high-frequency power value is reduced, it is sufficient to prolong an application time to enable processing.

The impedance detection circuit 21 according to this embodiment is constituted by using a differential circuit. In a differential output signal of an impedance which is output from this impedance detection circuit 21 and shown in FIG. 5D, a spark in abnormal discharge is represented as a pulsating signal having peaks generated between rising and falling (i.e., first and last parts in a section) peak parts of the high-frequency power. However, since a high-frequency noise component is also included, using a filter to remove such a component is preferable. The impedance detection circuit 21 may be formed by using an inverse function arithmetic circuit or a logarithmic amplifier, or a divider circuit or the like can be used.

FIGS. 5A to 5F show changes in a voltage waveform of the intermittently generated high-frequency power and an impedance. Specifically, FIG. 5A shows a voltage waveform of a normal high-frequency power, and abnormal discharge (a spark) is not produced. FIG. 5B shows a change in an impedance of a living tissue or a blood vessel held between the distal ends 2a of the sealing forceps 2 to which the high-frequency power is applied. This change is equivalent to that in FIG. 2B. FIG. 5C shows a change in the impedance when abnormal discharge occurs. FIG. 5D shows a differential output signal of the impedance in the impedance detection circuit 21. FIG. 5E shows output characteristics of the high-frequency power whose voltage is reduced by feedback control of the CPU 3 at the time of occurrence of abnormal discharge. FIG. 5F shows a change in the impedance of a held living tissue or a blood vessel involved by the high-frequency power whose voltage is reduced by feedback control of the CPU 3 at the time of occurrence of abnormal discharge.

A description will now be given as to output control of the thus configured electric processing system with reference to a flowchart shown in FIG. 6. It should be noted that operations in steps S21 to S24 and S27 to S32 in the flowchart of this embodiment are the same as those at steps S1 to S4 and S7 to S12 in the flowchart of FIG. 3, and hence corresponding steps will be briefly explained.

First, an operator instructs start of processing to commence application of a high-frequency power to the sealing forceps 2 and to also begin feedback control (steps S21 and S22). A judgment is made upon whether a voltage value of the high-frequency power applied to the sealing forceps 2 has reached a preset control voltage (e.g., 100 V: an upper limit value) (step S23). In this embodiment, rising of the high-frequency power is slightly inclined to avoid overshoot, thereby eliminating a factor of a reduction in quality of processing and start of abnormal discharge due to overshoot.

When it is determined that the high-frequency power has not reached the control voltage in the judgment at step S23 (NO), this application state is maintained. On the other hand, when the high-frequency power has reached the control voltage (YES), this control voltage is determined as a constant voltage and applied to the sealing forceps 2 as an intermittent output having a pulse waveform with an application time of approximately one second (step S24). At this time, an intermittent time (an output stop time) is set to approximately 500 milliseconds. These settings are the same as those in the first embodiment.

Subsequently, a detection signal obtained by differentiating a change in an impedance in the sealing forceps 2 is detected from the output signal of the high-frequency power by the impedance detection circuit 21 (step S25). Whether abnormal discharge (a spark) has been generated in the sealing forceps 2 is judged based on this detection signal (step S26). If it is determined that abnormal discharge has not been generated in this judgment (NO), application is continued with the set control voltage and intermittent output timing (step S27). On the other hand, when a pulsating signal is detected in such a differential signal of the impedance as shown in FIG. 5D and it is determined that abnormal discharge (a spark) has been generated, whether the current control voltage (a constant voltage) has been reduced to 80 V is judged. In the case of 80 V, the control advances to step S27 to continue application. In the case of 80 V or above, the control voltage is reduced by 10 V and the application time of one second is increased 10% to be prolonged to 1.1 seconds as in the first embodiment (steps S28 and S29).

Then, after steps S27 and S29, a judgment is made upon whether the preset application time (an intermittent output on time of approximately one second in this example) has been reached (step S30). When it is determined that the present application time has not been reached (NO), the control returns to step S25 and a change in the impedance is detected while continuing application of the high-frequency power. On the other hand, when the preset application time has been reached (YES), the output is stopped for the above-described output stop time of approximately 500 milliseconds (step S11).

Moreover, a judgment is made upon whether welding processing has been completed with respect to a blood vessel or the like (step S32). As to a timing of completion of this welding processing, it is sufficient to use a known completion judgment method, e.g., a history of an increase in a temperature in a periphery of a held part or attainment of a preset impedance value at the time of completion. If it is determined that the welding processing has not been completed yet in this judgment (NO), the control returns to step S22 to continue the processing sequence. On the other hand, if the welding processing has been completed (YES), this processing sequence is terminated.

As described above, according to the second embodiment, a differential output of the impedance of the intermittently generated high-frequency power is sequentially monitored for the feedback control with respect to the high-frequency power applied to the bipolar type sealing forceps 2. When abnormal discharge (a spark) is produced at the distal ends, the high-frequency power is reduced and the application time is extended. As a result, the abnormal discharge can be terminated, a reduction in quality of processing can be avoided, and end of processing can be readily judged.

## Claims

1. An electric processing system comprising:
a bipolar type forceps (2) adapted to receive a high-frequency power applied from the outside and to carry out processing to a living tissue held between distal ends;
a drive control apparatus (1) adapted to generate and output a high-frequency power which is intermittently applied to the forceps (2) by feedback control adapted to set an output value of a subsequent high-frequency power based on the precedently output high-frequency power; and
a control portion (3) adapted to sample the sequentially output high-frequency power, to detect a phase difference of a current and a voltage, to detect occurrence of abnormal discharge between the distal ends based on the phase difference, to reduce a voltage of the high-frequency power to an application voltage that has been empirically set as a voltage which stops abnormal discharge, to prolong an application time, and to use a completion judgment method configured to determine if the processing is completed; wherein the control portion (3) is provided in the drive control apparatus (1).

2. The electric processing system according to claim 1, wherein the completion judgment method comprises detection of one of: (i) an accumulated temperature of increases in temperature in a periphery of the held living body tissue part, (ii) an achievement of a preset impedance at the time of completion and (iii) whether a phase difference has reached a specified value.

3. The electric processing system according to claim 1 or 2, wherein the drive control apparatus (1) comprises a phase difference detection circuit (14) adapted to detect a change in the phase difference in the current and the voltage.

4. The electric processing system according to claim 3, wherein the phase difference detection circuit (14) comprises a zero cross circuit for detecting a change in the phase difference.

5. The electric processing system according to any one of claims 1 to 4, wherein the drive control apparatus (1) further comprises:
an output transformer (8) adapted to output the high-frequency power to the forceps (2);
an amplifier (7) adapted to amplify a high-frequency power supplied to the output transformer (8);
a detecting portion (9) adapted to measure a voltage value and a current value in the high-frequency power input to the output transformer (8);
an analog-to-digital conversion portion (12, 13) adapted to digitize the detected current value and voltage value;
a phase difference detection circuit (14) adapted to detect a phase difference of the current value and the voltage value output from the analog-to-digital conversion portion (12, 13);
the control portion (3);
a waveform generation circuit (6) adapted to generate a specified output waveform based on an instruction from the control portion (3); and
a power supply (4) adapted to generate the high-frequency power to be output based on an instruction from the control portion (3).

6. An electric processing system comprising:
a bipolar type forceps (2) adapted to receive a high-frequency power applied from the outside and to carry out processing to a living tissue held between distal ends;
a drive control apparatus (1) adapted to generate and output a high-frequency power which is intermittently applied to the forceps (2) by feedback control adapted to set an output value of a subsequent high-frequency power based on the precedently output high-frequency power; and
a control portion (3) adapted to sample the sequentially output high-frequency power, to detect a tissue impedance from measuring an output current and an output voltage, to detect occurrence of abnormal discharge between the distal ends of the forceps (2) based on presence/absence of a pulsating signal produced in a differential signal obtained by differentiating a detection signal of the impedance, to reduce a voltage of the high-frequency power to an application voltage that has been empirically set as a voltage which stops abnormal discharge, to prolong an application time, and to use a completion judgment method configured to determine if the processing is completed; wherein the control portion is provided in the drive control apparatus (1).

7. The electric processing system according to claim 6, wherein the completion judgment method comprises detection of one of: (i) a history of an increase in a temperature in a periphery of a held living tissue part and (ii) an attainment of a preset impedance value at the time of completion.

8. The electric processing system according to claim 6 or 7, wherein the drive control apparatus (1) comprises an impedance detection circuit (21) adapted to sample a current value and a voltage value of the generated high-frequency power and to detect an impedance.

9. The electric processing system according to any one of the claims 6 to 8, wherein the impedance detection circuit (21) comprises a differential circuit and can be formed by one of: an inverse function arithmetic circuit, a logarithmic amplifier and a divider circuit.

## Patentansprüche

1. Elektrisches Behandlungssystem, das umfasst:
eine in zweipoliger Bauart ausgebildete Zange (2), die dazu eingerichtet ist, eine von außen aufgebrachte Hochfrequenzenergie zu empfangen und ein lebendes Gewebe zu behandeln, das zwischen distalen Enden gehalten wird;
ein Antriebssteuerungsgerät (1), das dazu eingerichtet ist, eine Hochfrequenzenergie zu erzeugen und auszugeben, die periodisch an die Zange (2) angelegt wird durch eine Feedback-Steuerung, welche dazu eingerichtet ist, einen Ausgabewert einer nachfolgenden Hochfrequenzenergie basierend auf der vorher ausgegebenen Hochfrequenzenergie einzustellen; und
einen Steuerungsabschnitt (3), der dazu eingerichtet ist, die sequenziell ausgegebene Hochfrequenzenergie abzufragen, eine Phasendifferenz eines Stroms und einer Spannung zu erfassen, das Auftreten einer anormalen Entladung zwischen den distalen Enden basierend auf der Phasendifferenz zu erfassen, eine Spannung der Hochfrequenzenergie auf eine Anwendungsspannung zu reduzieren, die empirisch als eine Spannung festgelegt wurde, die eine anormale Entladung stoppt, eine Anwendungszeit zu verlängern und ein Beendigungsbeurteilungsverfahren zu benutzen, das dazu eingerichtet ist, zu bestimmen, ob die Behandlung beendet ist; wobei der Steuerungsabschnitt (3) in dem Antriebssteuerungsgerät (1) vorgesehen ist.

2. Elektrisches Behandlungssystem gemäß Anspruch 1, bei dem das Beendigungsbeurteilungsverfahren die Erfassung: (i) einer akkumulierten Temperatur von Temperaturerhöhungen in einer Umgebung des gehaltenen lebenden Körpergewebeteils, (ii) ein Erreichen einer vorbestimmten Impedanz zu dem Beendigungszeitpunkt oder (iii) ob eine Phasendifferenz einen bestimmten Wert erreicht hat, umfasst.

3. Elektrisches Behandlungssystem gemäß Anspruch 1 oder 2, bei dem das Antriebssteuerungsgerät (1) eine Phasendifferenzerfassungsschaltung (14) umfasst, die dazu eingerichtet ist, eine Änderung der Phasendifferenz des Strom oder der Spannung zu erfassen.

4. Elektrisches Behandlungssystem gemäß Anspruch 3, bei dem die Phasendifferenzerfassungsschaltung (14) eine Nulldurchgangsschaltung zum Erfassen einer Änderung der Phasendifferenz umfasst.

5. Elektrisches Behandlungssystem gemäß einem der Ansprüche 1 bis 4, bei dem das Antriebssteuerungsgerät (1) ferner umfasst:
einen Ausgabetransformator (8), der dazu eingerichtet ist, die Hochfrequenzenergie an die Zange (2) auszugeben;
einen Verstärker (7), der dazu eingerichtet ist, eine dem Ausgabetransformator (8) zugeführte Hochfrequenzenergie zu verstärken;
einen Erfassungsabschnitt (9), der dazu eingerichtet ist, einen Spannungswert und einen Stromwert in der in den Ausgabetransformator (8) eingegebenen Hochfrequenzenergie zu messen;
einen Analog-Digital-Wandler-Abschnitt (12, 13), der dazu eingerichtet ist, den erfassten Stromwert und den Spannungswert zu digitalisieren;
eine Phasendifferenzerfassungsschaltung (14), die dazu eingerichtet ist, eine Phasendifferenz des Stromwerts und des Spannungswerts, die von dem Analog-Digital-Wandler-Abschnitt (12, 13) ausgegeben werden, zu erfassen;
den Steuerungsabschnitt (3);
eine Wellenformerzeugungsschaltung (6), die dazu eingerichtet ist, basierend auf einer Anweisung des Steuerungsabschnitts (3) eine bestimmte Ausgabewellenform zu erzeugen; und
eine Energiequelle (4), die dazu eingerichtet ist, die zur Ausgabe basierend auf einer Anweisung des Steuerungsabschnitts (3) vorgesehene Hochfrequenzenergie zu erzeugen.

6. Elektrisches Behandlungssystem, das umfasst:
eine in zweipoliger Bauart ausgebildete Zange (2), die dazu eingerichtet ist, eine von außen angelegte Hochfrequenzenergie zu empfangen und ein lebendes Gewebe zu behandeln, das zwischen distalen Enden gehalten wird;
ein Antriebssteuerungsgerät (1), das dazu eingerichtet ist, eine Hochfrequenzenergie zu erzeugen und auszugeben, die periodisch an die Zange (2) angelegt wird durch eine Feedback-Steuerung, welche dazu eingerichtet ist, einen Ausgabewert einer nachfolgenden Hochfrequenzenergie basierend auf der vorher ausgegebenen Hochfrequenzenergie einzustellen; und
einen Steuerungsabschnitt (3), der dazu eingerichtet ist, die sequenziell ausgegebene Hochfrequenzenergie abzufragen, durch Messen eines Ausgabestroms und einer Ausgabespannung eine Gewebeimpedanz zu erfassen, das Auftreten einer anormalen Entladung zwischen den distalen Enden der Zange (2) basierend auf der Anwesenheit/Abwesenheit eines pulsierenden Signals zu erfassen, das in einem durch Differenzierung eines Erfassungssignals der Impedanz erhaltenen Differenzsignals erzeugt wird, eine Spannung der Hochfrequenzenergie auf eine Anwendungsspannung zu reduzieren, die empirisch als eine Spannung festgelegt wurde, die eine anormale Entladung stoppt, eine Anwendungszeit zu verlängern und ein Beendigungsbeurteilungsverfahren zu benutzen, das dazu eingerichtet ist, zu bestimmen, ob die Behandlung beendet ist; wobei der Steuerungsabschnitt (3) in dem Antriebssteuerungsgerät (1) vorgesehen ist.

7. Elektrisches Behandlungssystem gemäß Anspruch 6, bei dem das Beendigungsbeurteilungsverfahren die Ermittlung: (i) einer Historie einer Temperaturerhöhung in einer Umgebung eines gehaltenen lebenden Gewebeteils und (ii) ein Erreichen eines vorbestimmten Impedanzwerts zu dem Beendigungszeitpunkt, umfasst.

8. Elektrisches Behandlungssystem gemäß Anspruch 6 oder 7, bei dem das Antriebssteuerungsgerät (1) eine Impedanzerfassungsschaltung (21) umfasst, die dazu eingerichtet ist, einen Stromwert und einen Spannungswert der erzeugten Hochfrequenzenergie abzufragen und eine Impedanz zu erfassen.

9. Elektrisches Behandlungssystem gemäß einem der Ansprüche 6 bis 8, bei dem die Impedanzerfassungsschaltung (21) eine Differenzschaltung umfasst und durch eine inversfunktionsarithmetische Schaltung, einen logarithmischen Verstärker oder eine Teilerschaltung gebildet sein kann.

## Revendications

1. Système de traitement électrique comprenant :
une pince (2) de type bipolaire adaptée pour recevoir une puissance haute fréquence appliquée depuis l'extérieur et pour réaliser un traitement sur un tissu vivant tenu entre des extrémités distales ;
un appareil (1) de commande d'entraînement adapté pour générer et délivrer en sortie une puissance haute fréquence qui est appliquée par intermittence à la pince (2) par une commande de rétroaction adaptée pour établir une valeur de sortie d'une puissance haute fréquence suivante sur la base de la puissance haute fréquence délivrée en sortie précédemment ; et
une partie de commande (3) adaptée pour échantillonner la puissance haute fréquence délivrée en sortie séquentiellement, pour détecter une différence de phase d'un courant et d'une tension, pour détecter la survenue d'une décharge anormale entre les extrémités distales sur la base de la différence de phase, pour réduire une tension de la puissance haute fréquence à une tension d'application qui a été établie de manière empirique en tant que tension qui arrête une décharge anormale, pour prolonger un temps d'application, et pour utiliser un procédé de jugement d'achèvement configuré pour déterminer si le traitement est achevé ; dans lequel la partie de commande (3) est prévue dans l'appareil (1) de commande d'entraînement.

2. Système de traitement électrique selon la revendication 1, dans lequel le procédé de jugement d'achèvement comprend la détection de l'une parmi : (i) une température accumulée des augmentations de température dans une périphérie de la partie de tissu vivant tenue, (ii) une atteinte d'une impédance préétablie au moment de l'achèvement et (iii) si une différence de phase a atteint une valeur spécifiée.

3. Système de traitement électrique selon la revendication 1 ou 2, dans lequel l'appareil (1) de commande d'entraînement comprend un circuit (14) de détection de différence de phase adapté pour détecter un changement dans la différence de phase dans le courant et la tension.

4. Système de traitement électrique selon la revendication 3, dans lequel le circuit (14) de détection de différence de phase comprend un circuit de passage à zéro destiné à détecter un changement dans la différence de phase.

5. Système de traitement électrique selon l'une quelconque des revendications 1 à 4, dans lequel l'appareil (1) de commande d'entraînement comprend en outre :
un transformateur de sortie (8) adapté pour délivrer en sortie la puissance haute fréquence vers la pince (2) ;
un amplificateur (7) adapté pour amplifier une puissance haute fréquence délivrée vers le transformateur de sortie (8) ;
une partie de détection (9) adaptée pour mesurer une valeur de tension et une valeur de courant dans la puissance haute fréquence délivrée en entrée vers le transformateur de sortie (8) ;
une partie (12, 13) de conversion d'analogique en numérique adaptée pour numériser la valeur de courant et la valeur de tension détectées ;
un circuit (14) de détection de différence de phase adapté pour détecter une différence de phase de la valeur de courant et de la valeur de tension délivrées en sortie depuis la partie (12, 13) de conversion d'analogique en numérique ;
la partie de commande (3) ;
un circuit (6) de génération de forme d'onde adapté pour générer une forme d'onde de sortie spécifiée sur la base d'une instruction provenant de la partie de commande (3) ; et
une alimentation (4) adaptée pour générer la puissance haute fréquence destinée à être délivrée en sortie sur la base d'une instruction provenant de la partie de commande (3).

6. Système de traitement électrique comprenant :
une pince (2) de type bipolaire adaptée pour recevoir une puissance haute fréquence appliquée depuis l'extérieur et pour réaliser un traitement sur un tissu vivant tenu entre des extrémités distales ;
un appareil (1) de commande d'entraînement adapté pour générer et délivrer en sortie une puissance haute fréquence qui est appliquée par intermittence à la pince (2) par une commande de rétroaction adaptée pour établir une valeur de sortie d'une puissance haute fréquence suivante sur la base de la puissance haute fréquence délivrée en sortie précédemment ; et
une partie de commande (3) adaptée pour échantillonner la puissance haute fréquence délivrée en sortie séquentiellement, pour détecter une impédance de tissu à partir de la mesure d'un courant de sortie et d'une tension de sortie, pour détecter la survenue d'une décharge anormale entre les extrémités distales de la pince (2) sur la base de la présence/absence d'un signal impulsionnel produit dans un signal différentiel obtenu en différenciant un signal de détection de l'impédance, pour réduire une tension de la puissance haute fréquence à une tension d'application qui a été établie de manière empirique en tant que tension qui arrête une décharge anormale, pour prolonger un temps d'application, et pour utiliser un procédé de jugement d'achèvement configuré pour déterminer si le traitement est achevé ; dans lequel la partie de commande est prévue dans l'appareil (1) de commande d'entraînement.

7. Système de traitement électrique selon la revendication 6, dans lequel le procédé de jugement d'achèvement comprend la détection de l'un parmi : (i) un historique d'une augmentation de température dans une périphérie d'une partie de tissu vivant tenue et (ii) une atteinte d'une valeur d'impédance préétablie au moment de l'achèvement.

8. Système de traitement électrique selon la revendication 6 ou 7, dans lequel l'appareil (1) de commande d'entraînement comprend un circuit (21) de détection d'impédance adapté pour échantillonner une valeur de courant et une valeur de tension de la puissance haute fréquence générée et pour détecter une impédance.

9. Système de traitement électrique selon l'une quelconque des revendications 6 à 8, dans lequel le circuit (21) de détection d'impédance comprend un circuit différentiel et peut être formé par l'un parmi : un circuit arithmétique de fonction inverse, un amplificateur logarithmique et un circuit diviseur.
